# EUROPÄISCHE PATENTSCHRIFT

(11) **EP 0 859 759 B1**
(45) Veröffentlichungstag und Bekanntmachung des Hinweises auf die Patenterteilung: **24.07.2002**
(21) Anmeldenummer: 96934541.2
(22) Anmeldetag: 04.10.1996
(51) Int. Cl.: C07D 209/08, A61K 7/42

(54) **SULFONSÄUREN UND IHRE VERWENDUNG ALS UV-ABSORBER**
SULPHONIC ACIDS AND THEIR USE AS U/V ABSORBERS
ACIDES SULFONIQUES ET LEUR UTILISATION COMME ABSORBEURS DES UV

(30) Priorität: 16.10.1995 DE 19539623
(43) Veröffentlichungstag der Anmeldung: 26.08.1998
(73) Patentinhaber: HAARMANN & REIMER GMBH, D-37601 Holzminden (DE)
(72) Erfinder: KOCH, Oskar, D-37079 Göttingen (DE); LANGNER, Roland, D-37639 Bevern (DE); KREMPEL, Alfred, D-37603 Holzminden (DE)
(74) Vertreter: Petrovicki, Wolfgang, Dr.
(86) Internationale Anmeldenummer: EP9604325
(87) Internationale Veröffentlichungsnummer: WO9714680

(56) Entgegenhaltungen:
- EP-A- 0 207 287
- DE-A- 1 568 541
- DE-A- 4 406 024
- DE-C- 4 203 072
- US-A- 4 250 315

## Beschreibung

Die Erfindung betrifft neue Sulfonsäuren, ein Verfahren zu ihrer Herstellung und die Verwendung als UV-Absorber, insbesondere in Sonnenschutzmitteln.

UV-Strahlen werden je nach Wellenlänge als UV-A-Strahlen (320-400 nm, UV-A-I: 340-400 nm, UV-A-II: 320-340 nm) oder UV-B-Strahlen (280-320 nm) bezeichnet. Ganz allgemein gilt: Die schädigende Wirkung der UV-Strahlen auf die menschliche Haut steigt mit sinkender Wellenlänge und steigender Dauer der Exposition.

So können UV-Strahlen Hautschädigungen hervorrufen, wobei die UV-B-Strahlung einen Sonnenbrand (Erythem) bis hin zu schwersten Hautverbrennungen verursachen kann. Sehr häufige und ungeschützte Bestrahlung der Haut mit Sonnenlicht führt auch zu einem Verlust der Hautelastizität und zu vermehrter Faltenbildung, insgesamt zu frühzeitiger Alterung der Haut. In extremen Fällen können krankhafte Hautveränderungen bis hin zum Hautkrebs auftreten.

Die UV-A-Strahlung bewirkt eine rasche, schwache Direktpigmentierung der Haut. UV-A-Strahlen dringen in tiefere Hautschichten ein und können dort den Alterungsprozeß der Haut beschleunigen. Die kürzerwellige W-A-II-Strahlung unterstützt die Bildung von Sonnenbrand. Weiterhin kann die UV-A-Strahlung phototoxische oder photoallergische Hautreaktionen auslösen. Es existieren gesicherte Zusammenhänge zwischen UV-A-Exposition und erhöhtem Hautkrebsrisiko.

Entsprechend der Lage ihrer Absorptionsmaxima werden UV-Absorber für kosmetische und pharmakologische Präparate in UV-A- und UV-B-Absorber eingeteilt.

Aus toxikologischen Gründen sollen heutzutage UV-Absorber, die auf die menschliche Haut aufgetragen werden, die Haut möglichst nicht durchdringen.

Als UV-Absorber sind bereits die verschiedensten Verbindungen, wie z.B. Phenylbenzimidazolsulfonsäure (DE-OS 42 03 072), 2-Hydroxy-4-methoxybenzophenon-5-sulfonsäure (EP-A 557 089) und Terephthalyliden-dibornan-sulfonsäure (DE-OS 33 21 679) vorgeschlagen worden. Diese Verbindungen absorbieren entweder nicht die gewünschte Wellenlänge oder besitzen nur eine geringe Absorption oder sind wegen der notwendigen Rohstoffe oder der nicht ergiebigen Herstellverfahren nicht so zugänglich, wie es wünschenswert wäre.

EP-A 207 287 und DE-A 15 68 541 offenbaren Indolinderivate, die sich als UV-Absorber eignen.

Der Erfindung liegt deshalb die Aufgabe zugrunde, verbesserte UV-Absorber zur Verfügung zu stellen.

Die Erfindung betrifft Verbindungen der Formel 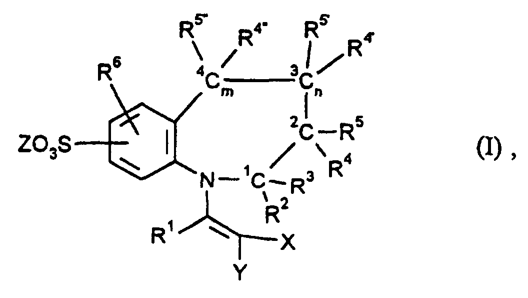 worin
- R¹ bis R⁵: (einschließlich R^{4'}, R^{4"}, R^{5'}, R^{5"}) unabhängig voneinander Wasserstoff, C₁-C₈-Alkyl oder C₅-C₁₀-Cycloalkyl bedeuten mit der Maßgabe, daß auch zwei Substituenten an benachbarten C-Atomen aus der Reihe ¹C bis ⁴C zusammen eine gegebenenfalls substituierte Polymethylengruppe, insbesondere C₃-C₄-Alkylen bedeuten können, wobei eine Methylengruppe durch -O-, -S- oder -NH- ersetzt sein kann,
- R⁶: Wasserstoff, C₁-C₈-Alkyl, C₅-C₁₀-Cycloalkyl, Hydroxy, C₁-C₈-Alkoxy, COOR⁶⁰, CONR⁶¹R⁶²,
- R⁶⁰ bis R⁶²: unabhängig voneinander Wasserstoff oder C₁-C₆-Alkyl,
- X, Y: unabhängig voneinander Wasserstoff, CN, CO₂R¹⁰, CONR¹⁰R¹¹, COR¹⁰
wobei einer der Reste X oder Y zusätzlich ein C₁-C₈-Alkylrest, ein C₅-C₁₀-Arylrest, insbesondere Phenyl, oder ein Heteroarylrest mit 5 bis 6 Ringatomen (davon 1 bis 2 Heteroatome aus der Reihe N, O, S) sein kann,
wobei ferner X und Y oder
R¹ zusammen mit einem der Reste X und Y den Rest zur Vervollständigung eines 5 bis 7-gliedrigen Ringes bedeuten kann, der bis zu 3 Heteroatome, insbesondere Sauerstoff und/oder Stickstoff, enthalten kann, wobei die Ringatome substituiert sein können (insbesondere mit exocyclisch doppelt gebundenem Sauerstoff (Keto-Sauerstoff) und/oder C₁-C₈-Alkyl- und/oder C₅-C₁₀-Cycloalkylresten) und/oder C=C-Doppelbindungen enthalten können,
- Z: Wasserstoff, Ammonium, Alkalimetallion, insbesondere Lithium, Natrium Kalium, 1/2 Äquivalente Erdalkalimetallion, vorzugsweise Calcium, Magnesium oder das Kation einer zur Neutralisation der freien Säuregruppe eingesetzte organische Stickstoffbase,
- R¹⁰, R¹¹: unabhängig voneinander Wasserstoff, Alkyl, insbesondere C₁-C₈-Alkyl oder Cycloalkyl, insbesondere mit 5 bis 10 Ringatomen, und
- n, m: unabhängig voneinander Null oder 1 bedeuten.

Die erfindungsgemäßen Sulfonsäuren eignen sich besonders gut für die Verwendung in Sonnenschutzmitteln, vorzugsweise in kosmetischen und pharmakologischen Präparaten. Sie zeichnen sich durch eine sehr hohe Absorption, eine ausgezeichnete Lichtstabilität und durch eine sehr geringe Tendenz zur Hautpenetration aus.

Bevorzugte Verbindungen I sind solche, worin R¹ bis R⁶ (einschließlich R^{4'}, R^{4"}, R^{5'} und R^{5"}) Wasserstoff bedeuten und n und m Null sind.

Weitere bevorzugte Verbindungen I sind solche, in denen X Cyan und Y Carb-C₁-C₄alkoxy bedeuten.

Weitere bevorzugte Verbindungen I sind solche, bei denen die Substituenten X und Y zusammen mit dem Kohlenstoffatom, an dem sie sitzen, ein 2-Methyl-4H-oxazol-5-on, ein Imidazolidin-2.4-dion oder Cyclopentanon bedeuten.

Besonders bevorzugte Verbindungen I entsprechen den Formeln

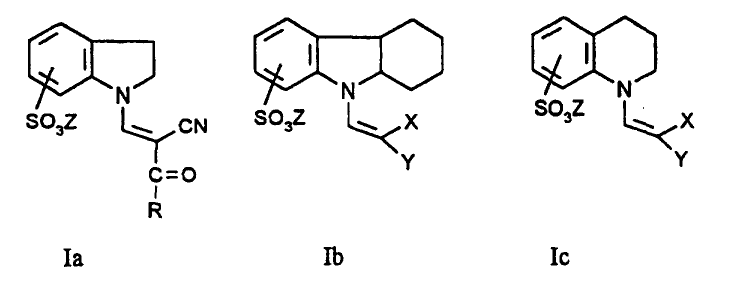

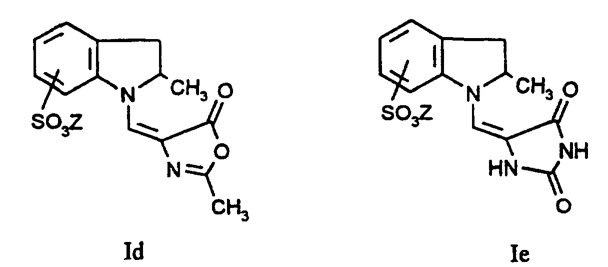

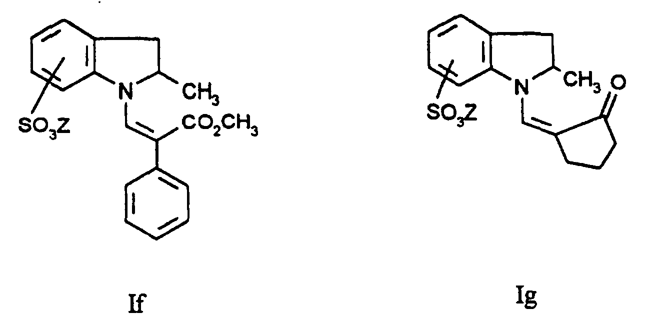

Die Verbindungen (I) können durch Sulfonierung der Verbindungen (II) 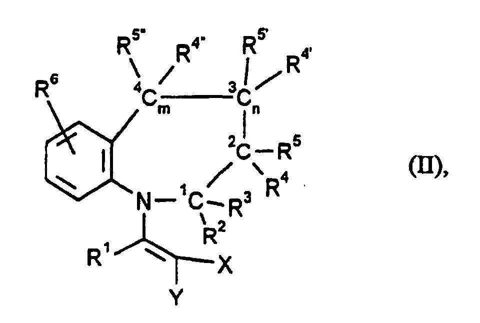 worin die Substituenten und Indices die oben angegebene Bedeutung besitzen, erhalten werden.

Die Verbindungen II und Verfahren zu ihrer Herstellung sind teilweise bekannt (DE-OS 1 568 541, 3 519 926); soweit sie nicht bekannt sind, können die Verbindungen II analog den bekannten Verfahren hergestellt werden. In der Regel wird das entsprechende cyclische sekundäre Amin mit einer C-H-aciden Komponente und einem Carbonsäureorthoester kondenisert.

Die Sulfonierung kann mit üblichen Sulfonierungsmitteln erfolgen. Zu diesen zählen beispielsweise Schwefelsäure, Schwefeltrioxid, Oleum (vorzugsweise mit SO₃-Gehalten von 5 bis 30 Gew.-%), Chlorsulfonsäure und - vorzugsweise -Schwefelsäure und Schwefelsäure/Acetanhydrid-Mischungen.

Die Umsetzung kann bei Temperaturen von 0 bis 200, vorzugsweise 20 bis 120, insbesondere 30 bis 80°C, durchgeführt werden. Für die Reaktion können die Sulfonierungsmittel in stöchiometrisch errechneten Mengen oder im Überschuß eingesetzt werden; sofern man nicht mit einem großen Überschuß an Sulfonierungsmittel arbeiten will, betragen die Mengen Sulfonierungsmittel im allgemeinen 1 bis 4, vorzugsweise 1 bis 2 Äquivalente, bezogen auf zu sulfonierendes Indolin II. Man kann auch in nicht-sulfonierenden Lösungsmitteln arbeiten; dabei kommen auch solche organischen Lösungsmittel in Frage, die mit Wasser nicht mischbar sind und als Schleppmittel für Wasser wirken, wie z.B. Methylenchlorid, Chloroform, Toluol, Ligroin etc.

Besonders schonend verläuft die Sulfonierung in Lösungsmitteln, so z.B. mit Schwefelsäure oder Schwefeltrioxid in Chloroform oder flüssigem Schwefeldioxid, mit Schwefelsäure/Acetanhydrid in Essigsäure oder mit Chlorsulfonsäure in Chloroform.

Bei der Sulfonierung mit Chlorsulfonsäure hat man grundsätzlich die Wahl, ob man durch Umsatz mit einer äquimolaren Menge Chlorsulfonsäure direkt die Bildung der Indolinsulfonsäure I oder mit 2 Mol Chlorsulfonsäure pro Mol Indolin die Bildung von Indolinsulfochlorid anstrebt, aus dem dann die Indolinsulfonsäure durch Hydrolyse freigesetzt werden kann.

Für die Herstellung der Verbindung I (X = CN) setzt man nach einer besonderen Ausführungsform Verbindung II mit Schwefelsäure/Acetanhydrid in Essigsäure um, was dazu führt, daß die entstandenen Sulfonsäuren ausfallen. Man kann diese dann abtrennen und so eine wäßrige Aufarbeitung und den damit verbundenen Anfall von Abwässern vermeiden. Wenn man Verfahrensvarianten wählt, bei denen das gewünschte Produkt nicht ausfällt, muß hydrolysiert werden; das Produkt kann dann durch Eindampfen der wäßrigen Phase gewonnen werden.

Weiterer Gegenstand der Erfindung ist also ein Verfahren zur Herstellung der Verbindungen I durch Umsetzung der Verbindung II mit Sulfonierungsmitteln.

Es war überraschend, daß durch die Sulfonierung die Absorptionsmaxima nach höheren Wellenlängen verschoben werden, so daß die erfindungsgemäßen Produkte I Absorptionsmaxima von etwa 340 nm aufweisen - also gerade an der Grenze zwischen UV-A-I und UV-A-II.

Weiterer Gegenstand der Erfindung ist die Verwendung der Verbindungen I als UV-Absorber, vorzugsweise in Sonnenschutzmitteln.

Diese UV-Absorber besitzen eine glückliche Kombination wünschenswerter Eigenschaften, nämlich
- ausgezeichnete Lichtstabilität,
- toxikologische und dermatologische Unbedenklichkeit,
- ausgezeichnete Thermostabilität,
- sehr gute Löslichkeit in kosmetischen Lösungsmitteln (Öle, Wasser, Glykole, Alkohol usw.),
- Verträglichkeit mit kosmetischen Grundstoffen,
- pH-Stabilität,
- problemlose Verarbeitbarkeit in kosmetischen Formulierungen und Stabilität unter Anwendungsbedingungen,
- Verträglichkeit mit Verpackungsmaterialien,
- keine Färbung von Textilien bzw. Flecken müssen problemlos auswaschbar sein,
- Farblosigkeit und Geruchsneutralität.

Hervorzuheben ist, daß kosmetische und pharmazeutische Zubereitungen mit den neuen UV-Absorbern auch mit niedrigen pH-Werten stabil formuliert werden können, ohne daß eine Auskristallisation eintritt.

Die erfindungsgemäßen Verbindungen können als UV-A-Absorber in kosmetischen oder pharmazeutischen Zubereitungen verwendet werden, die den Durchtritt der UV-Strahlen durch den aufgetragenen Film der Zubereitung verhindern. Dies ist im allgemeinen dann der Fall, wenn die kosmetischen bzw. pharmazeutischen Zubereitungen 0,5 bis 15, vorzugsweise 1 bis 10, insbesondere 2 bis 5 Gew.-% (bezogen auf das Gesamtgewicht der Zubereitung) der erfindungsgemäßen Verbindungen enthalten.

Die die erfindungsgemäßen Verbindungen enthaltenden Zubereitungen können zum Schutz der Haut und der Haare - insbesondere durch Dauerwelle, Färbung und Bleichung bereits vorgeschädigter Haare - vor UV-Bestrahlung verwendet werden. Diese zum Schutz der Haut vor der UV-Strahlung dienenden kosmetischen und pharmazeutischen Zubereitungen können in den üblicherweise verwendeten Anwendungsformen vorliegen, d.h. als Öl-in-Wasser- oder Wasser-in-Öl-Emulsion, als Milch, als Lotion oder Creme, wäßrig oder wäßrig-alkoholisches Gel oder Lotion, Aerosol, Hydrodispersions-Gel (emulgatorfrei) oder jegliche andere übliche kosmetische oder pharmazeutische Zubereitung. Für den Schutz der Haare vor UV-Strahlen werden bevorzugt Zubereitungen als Shampoo, Spülung, Kur, Gel, Lotion, Spray oder Creme verwendet.

Die kosmetischen und pharmazeutischen Zubereitungen können die in diesen Mitteln üblicherweise verwendeten Bestandteile wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropylpalmitat, Isooctylstearat, Adipinsäurediisopropylester usw.), natürliche oder synthetische Öle oder Wachse, Pigmente (z.B. Titandioxid, Zinkoxid, Perglanzpigmente, Farbpigmente), Verdickungsmittel (z.B. Hydroxyethylcellulose, Bentonit usw.), Konservierungsstoffe, Feuchtigkeitsmittel, Vitamine, Siliconöle, Glycerin, Ethylalkohol, Parfumöle enthalten.

Die erfindungsgemäßen Verbindungen können einzeln oder in Mischung in den entsprechenden Zubereitungen eingesetzt werden; man kann sie auch in Kombination mit anderen UV-Absorbern - insbesondere UV-B-Absorbern zur Erzielung einer UV-A/B-Breitbandabsorption, oder mit nicht lichtstabilen UV-Absorbern (z.B. Butyl-methoxydibenzoyl-methan, 4-Isopropyldibenzoylmethan, p-Dimethylaminobenzoesäure-2-ethylhexylester, p-Methoxyzimtsäure-2-ethylhexylester und -iso-amylester) zu deren Stabilisierung einsetzen. Beispiele für solche Verbindungen umfassen:
p-Aminobenzoesäure
p-Aminobenzoesäureethylester (25 Mol) ethoxyliert
p-Dimethylaminobenzoesäure-2-ethylhexylester
p-Aminobenzoesäureethylester (2 Mol) N-propoxyliert
p-Aminobenzoesäureglycerinester
Salicylsäure-homomenthylester
Salicylsäure-2-ethylhexylester
Triethanolaminsalicylat
4-Isopropylbenzylsalicylat
Anthranilsäurementhylester
Diisopropylzimtsäureethyl ester
p-Methoxyzimtsäure-2-ethylhexylester
Diisopropylzimtsäuremethylester
p-Methoxyzimtsäureisoamylester
p-Methoxyzimtsäure-diethanolaminsalz
p-Methoxyzimtsäure-isopropylester
2-Ethylhexyl-2-cyano-3,3-diphenylacrylat
Ethyl-2-cyano-3,3'-diphenylacrylat
2-Phenylbenzimidazolsulfonsäure und Salze
N,N,N-Trimethyl-4-(2-oxoborn-3-ylidenmethyl)-aniliniummethylsulfat
Tetraphthalyliden-dibornansulfonsäure und Salze
4-t-Butyl-4'-methoxy-dibenzoylmethan
β-Imidazol-4(5)-acrylsäure (Urocaninsäure)
2-Hydroxy-4-methoxybenzophenon
2-Hydroxy-4-methoxybenzophenon-5-sulfonsäure
Dihy droxy-4-methoxybenzophenon
2,4-Dihydroxybenzophenon
Tetrahydroxybenzophenon
2,2'-Dihydroxy-4,4'-dimethoxybenzophenon
2-Hydroxy-4-n-octoxybenzophenon
2-Hydroxy-4-methoxy-4'-methylbenzophenon
α-(2-Oxobom-3-yliden)-tolyl-4-sulfonsäure und Salze
3-(4'-Methylbenzyliden)-d,1-campher
3-Benzyliden-d,1-campher
4-Isopropyldibenzoylmethan
2,4,6-Trianilino-(p-carbo-2'-ethylhexyl-1'-oxy)-1,3,5-triazin.

Besonders geeignete UV-B-Absorber sind:
p-Methoxyzimtsäure-2-ethylhexylester,
p-Methoxyzimtsäure-isoamylester,
2-Phenylbenzimidazolsulfonsäure,
3-(4'-Methylbenzyliden)-d,1-campher,
2-Ethylhexyl-2-cyano-3,3-diphenylacrylat und
Salicylsäure-2-ethylhexylester.

Ebenso ist die Kombination der Verbindungen I mit feinteiligen Pigmenten, wie z.B. Titandioxid, Zinkoxid und Eisenoxid, in Sonnenschutz- und Tagespflegeprodukten mit UV-Schutz möglich.

### Beispiele

### Beispiel 1

### N-(β-Cyano-β-carbomethoxy-vinyl)-2-methylindolin-5-sulfonsäure

Eine Mischung aus 108 g (1,10 Mol) konz. Schwefelsäure und 112 g (1,10 Mol) Acetanhydrid wurde in einer Rührapparatur vorgelegt und innerhalb von 15 min mit einer Lösung von 134 g (0,55 Mol) N-(β-Cyano-β-carbmethoxy-vinyl)-2-methylindolin in 400 g Essigsäure bei 30 bis 40°C versetzt. Man rührte noch ca. 15 bis 20 min nach, wobei das Produkt aus dem Reaktionsgemisch ausfiel. Nach Abfiltrieren wurde aus einem Gemisch Isopropanol/Wasser (3 : 1 Volumenteile) umkristallisiert; man erhielt nach Trocknung (lh/50°C) 186 g Produkt entsprechend einer Ausbeute von 95 %. Schmelzpunkt >270°C, λmax = 339 nm, E1/1 = 1140.

### Beispiele 2 und 3

Die nachfolgenden Verbindungen wurden analog zu Beispiel 1 hergestellt.
- 2.: N-(β-β-Dicarbmethoxy-vinyl)-2-methylindolin-5-sulfonsäure λmax = 338 nm, E1/1 = 1 100
- 3.: N-(β-Acetyl-β-carbomethoxy-vinyl)-2-methylindolin-5-sulfonsäure λmax = 350 nm, E1/1 =1 050

In den folgenden Beispielen wurden die Verbindungen gemäß Beispiel 1, 2 oder 3 verwendet.

### Verwendete Komponenten:

- 30 DF:: Aluminiumdistearat, Lieferant 14
- Arlacel 165:: Glycerinstearat/Polyethylenglykol(M4G100)-stearat-Mischung, Lieferant 4
- Arosol:: Phenoxyethanol, Lieferant 1
- Betone Gel MIO:: Mineralöl, Lieferant 10
- Carbopol 941:: Polyacrylsäure, Lieferant 2
- Carbopol ETD 2001:: Acrylsäure-Copolymerisat, Lieferant 2
- Cetiol OE:: Dicaprylether, Lieferant 3
- Cetiol SN:: Cetyl-/Stearyl-isononanoat, Lieferant 3
- Cremophor NP 14:: mit 14 Mol Ethylenoxid verethertes Nonylphenol, Lieferant 6
- Cutina CBS:: Glycerinstearat, Cetyl-/Stearylalkohol, Cetylpalmitat, Kokosnußglyceride, Lieferant 3
- Cutina FS 45:: Palmitin-/Stearinsäure-Mischung, Lieferant 3
- Cutina MD:: Glycerinstearat, Lieferant 3
- Dehyquart A:: Cetyltrimethylammoniumchlorid, Lieferant 3
- Eumulgin B 1:: Cetyl-/Stearylalkohol, verethertmit 12 Mol Ethylenoxid, Lieferant 3
- Eumulgin B 2:: Cetyl-/Stearylalkohol, verethert mit 20 Mol Ethylenoxid, Lieferant 3
- Heliopan, Typ AV: .: p-Methoxyzimtsäureisooctylester, Lieferant 1
- Heliopan, Typ E 1000:: p-Methoxyzimtsäureisoamylester, Lieferant 1
- Lanette O:: Cetyl-/Stearylalkohol-Mischung, Lieferant 3
- Mulsifan RT 203/80:: Fettalkoholpolyglykolether, Lieferant 7
- Myritol 318:: Capryl-/Caprin-triglycerid, Lieferant 3
- Natrosol 250 HHR:: Hydroxyethylcellulose, Lieferant 12
- Neo Heliopan, Typ BB:: 2-Hydroxy-4-methoxybenzophenon, Lieferant 1
- Nutrilan L:: Eiweißhydrolysat, Na-salz, Lieferant 3
- Phenonip:: Gemisch von p-Hydroxybenzoesäureestern, Lieferant 9
- Quaternium-18 Hectorit:: Propylencarbonat, Lieferant 10
- Solbrol P:: p-Hydroxybenzoesäurepropylester, Lieferant 5
- Solbrol M:: p-Hydroxybenzoesäuremethylester, Lieferant 5
- Neo Heliopan, Typ MBC: 4-Methylbenzyliden-campher, Lieferant 1
- Zinkoxid neutral: Zinkoxid, Lieferant 1
- Neo Heliopan, Typ OS: Octylsalicylat, Lieferant 1
- Neo Heliopan, Typ MA: Methylanthranilat, Lieferant 1
- Uvinul P 25:: Polyethylenglykolester der p-Aminobenzoesäure, Lieferant 6
- Veegum Ultra:: Magnesiumaluminiumsilikat. Lieferant 11

### Lieferanten

1. Haarmann & Reimer GmbH, Holzminden
2. B.F. Goodrich Comp., Neuss
3. Henkel KGaA, Düsseldorf
4. ICI Speciality Chemicals, Frankfurt
5. Bayer AG, Leverkusen
6. BASF, Ludwigshafen
7. Zschimmer & Schwarz GmbH, Lahnstein
8. Nipa Lab. Ltd., Pontypridd Mid Glam, Wales, GB
9. Schülke & Mayr GmbH, Norderstedt
10. R.T. Vanderbilt Company Inc., Norwalk, USA
11. Hercules Inc., Wilmington, Del, USA

### Beispiel 4

Eine Sonnenschutzlotion (O/W) folgender Zusammensetzung wurde hergestellt:

| | Bestandteile | % |
|---|---|---|
| A) | Cutina FS 45 | 2,00 |
| | Eumulgin B 1 | 0,25 |
| | Eumulgin B 2 | 0,25 |
| | Cutina MD | 2,00 |
| | Lanette O | 2,80 |
| | Myritol 318 | 5,00 |
| | Paraffinöl 65 cp | 3,00 |
| | Arosol | 0,80 |
| | Solbrol P | 0,10 |
| | p- Methoxyzimtsäureisooctylester | 3,00 |
| | p-Methoxyzimtsäureisoamylester | 3,00 |
| | Neo Heliopan, Typ BB | 1,50 |
| | | |
| B) | Wasser, dest. | 67,05 |
| | Carbopol 941 | 0,30 |
| | Natriumhydroxid, 10 %ig in Wasser | 2,45 |
| | 1,2-Propylenglykol | 2,00 |
| | Solbrol M | 0,20 |
| | UV-A-Absorber gemäß Beispiel 1 | 4,00 |
| | | |
| C) | Parfumöl | 0,30 |

### Herstellung:

Teil A wurde bei 75 bis 80°C aufgeschmolzen.

Zur Herstellung des Teiles B wurde Carbopol klumpenfrei im Wasser dispergiert, mit Natronlauge dispergiert. Anschließend wurden die restlichen Inhaltsstoffe zugegeben und auf ca. 95°C erhitzt.

Anschließend wurde Teil B unter Rühren zu Teil A gegeben und auf Raumtemperatur abgekühlt. Bei ca. 30°C wurde Teil C zugegeben.

### Beispiel 5

Eine Sonnenschutzlotion (O/W) folgender Zusammenstellung wurde hergestellt:

| | Bestandteile | % |
|---|---|---|
| A) | Arlacel 165 | 3,00 |
| | Eumulgin B 2 | 1,00 |
| | Lanette O | 2,00 |
| | Myritol 318 | 4,00 |
| | Cetiol OE | 6,00 |
| | Betone Gel MIO und Quaternium-18 Hectorite | 3,00 |
| | Phenonip | 0,20 |
| | Cutina CBS | 2,00 |
| | p-Methoxyzimtsäureisooctylester | 7,00 |
| | 4-Methylbenzyliden-campher | 1,00 |
| | Zinkoxid neutral | 5,00 |
| | | |
| B) | Wasser, dest. | 57,90 |
| | Veegum Ultra | 1,00 |
| | Natrosol 250 HHR | 0,30 |
| | Glycerin 85 % | 3,00 |
| | Phenonip | 0,30 |
| | UV-A-Absorber gemäß Beispiel 1 | 3,00 |
| | | |
| C) | Parfumöl | 0,30 |

### Herstellung:

Zur Herstellung des Teiles A wurden die Komponenten bei 80°C aufgeschmolzen, zusammengegeben und Zinkoxid unter Dispergieren hinzugefügt.

Zur Herstellung des Teiles B wurden die Komponenten ohne Veegum und Natrosol auf 90°C erhitzt, dann diese Komponenten unter Dispergieren hinzugegeben. Anschließend wurde Teil B unter Rühren zu Teil A gegeben und auf Raumtemperatur abgekühlt. Dann wurde Teil C bei 30°C zugegeben und anschließend homogenisiert bei einem pH-Wert von 7,0-7,5.

### Beispiel 6

Eine Sonnenschutzcreme (O/W) folgender Zusammensetzung wurde hergestellt:

| | Bestandteile | % |
|---|---|---|
| A) | Cutina FS 45 | 2,00 |
| | Eumulgin B 1 | 0,25 |
| | Eumulgin B2 | 0,25 |
| | Cutina MD | 2,00 |
| | Lanette O | 3,00 |
| | Myritol 318 | 5,00 |
| | Cetiol SN | 3,00 |
| | Arosol | 0,80 |
| | Solbrol P | 0,10 |
| | p-Methoxyzimtsäureisoamylester | 5,00 |
| | p-Methoxyzimtsäureisooctylester | 3,00 |
| | 4-Methylbenzyliden-campher | 1,00 |
| | Octylsalicylat | 3,00 |
| | Butylmethoxydibenzoylmethan | 1,00 |
| | | |
| B) | Wasser dest. | 60,90 |
| | Carbopol 940 | 0,40 |
| | Natriumhydroxid, 10%ig in Wasser | 1,80 |
| | 1,2-Propylenglykol | 2,00 |
| | Solbrol M | 0,20 |
| | UV-A-Absorber gemäß Beispiel 1 | 5,00 |
| | | |
| C) | Parfumöl | 0,30 |

### Herstellung:

Die Komponenten des Teiles A wurden bei 80°C aufgeschmolzen und zusammengegeben. Zur Herstellung des Teiles B wurde Carbopol klumpenfrei in Wasser dispergiert, anschließend die restlichen Bestandteile zugegeben und auf ca. 90°C erhitzt. Anschließend wurde Teil B unter Rühren zu Teil A gegeben und auf Raumtemperatur abgekühlt. Der Zusatz des Teiles C erfolgte bei 30°C.

### Beispiel 7

Ein Sonnenschutzgel folgender Zusammensetzung wurde hergestellt:

| | Bestandteile | % |
|---|---|---|
| A) | Ethylalkohol | 5,00 |
| | Wasser, dest. | 64,60 |
| | 1,2-Propylenglykol | 5,00 |
| | D-Panthenol | 0,50 |
| | Carbopol ETD 2001 | 1,10 |
| | | |
| B) | Wasser | 5,00 |
| | Triethanolamin | 2,30 |
| | | |
| C) | Neo Heliopan, Typ Hydro, eingesetzt als 30%ige Lösung nach Neutralisation mit Triethanolamin / Phenylbenzimidazolsulfonsäure; 10.00 % entspricht Aktivsubstanz: 3,00 % | 10,00 |
| | UV-A-Absorber gemäß Beispiel 1 | 4,00 |
| | | |
| D) | Cremophor NP 14 | 1,20 |
| | Parfumöl | 0,30 |

### Herstellung:

Zur Herstellung des Teiles A wurden die einzelnen Bestandteile in Wasser gelöst, zusammengegeben und Carbopol klumpenfrei unter Dispergieren hinzugefügt. Anschließend wurde Triethanolamin (Teil B) in Wasser gelöst und unter Rühren zu Teil A hinzugefügt. Danach wurde Teil C und dann Teil D unter Rühren hinzugefügt.

### Beispiel 8

Eine Leave-on-Haarkur folgender Zusammensetzung wurde hergestellt:

| | Bestandteile | % |
|---|---|---|
| A) | Wasser, dest. | 83,85 |
| | Natrosol 250 HHR | 0,70 |
| | Ethylalkohol | 5,00 |
| | Uvinul P 25 | 5,00 |
| | | |
| | UV-A-Absorber gemäß Beispiel 1 | 1,00 |
| | | |
| B) | Nutrilan L | 2,00 |
| | Dehyquart A | 0,20 |
| | Phenonip | 0,50 |
| | Triethanolamin | 0,25 |
| | | |
| C) | Mulsifan RT 203/80 | 1,20 |
| | Parfümöl | 0,30 |

### Herstellung:

Zur Herstellung des Teiles A wurde Wasser auf ca. 85°C erhitzt, Natrosol eingestreut und unter starkem Rühren auf Raumtemperatur abgekühlt. Anschließend wurden die restlichen Bestandteile des Teiles A, dann die Teile B und dann die Teile B und C hinzugefügt.

## Patentansprüche

1. Die Erfindung betrifft Verbindungen der Formel 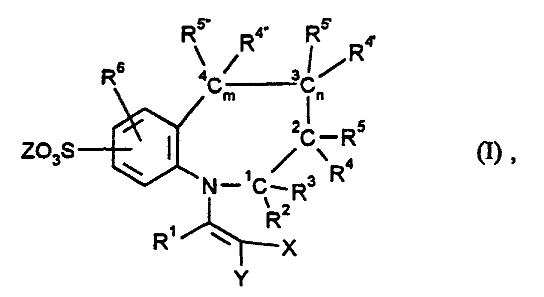 worin
R¹ bis R⁵ (einschließlich R^{4'}, R^{4"}, R^{5'}, R^{5"}) unabhängig voneinander Wasserstoff, C₁-C₈-Alkyl oder C₅-C₁₀-Cycloalkyl bedeuten mit der Maßgabe, daß auch zwei Substituenten an benachbarten C-Atomen aus der Reihe ¹C bis ⁴C zusammen eine gegebenenfalls substituierte Polymethylengruppe bedeuten können, wobei eine Methylengruppe durch -O-, -S- oder -NH- ersetzt sein kann,
R⁶ Wasserstoff, C₁-C₈-Alkyl, C₅-C₁₀-Cycloalkyl, Hydroxy, C₁-C₈-Alkoxy, COOR⁶⁰, CONR⁶¹R⁶²,
R⁶⁰ bis R⁶² unabhängig voneinander Wasserstoff oder C₁-C₆-Alkyl,
X, Y unabhängig voneinander Wasserstoff, CN, CO₂R¹⁰, CONR¹⁰R¹¹, COR¹⁰
wobei einer der Reste X oder Y zusätzlich ein C₁-C₈-Alkylrest, ein C₅-C₁₀-Arylrest oder ein Heteroarylrest mit 5 bis 6 Ringatomen (davon 1 bis 2 Heteroatome aus der Reihe N, O, S) sein kann,
wobei ferner X und Y oder
R¹ zusammen mit einem der Reste X und Y den Rest zur Vervollständigung eines 5 bis 7-gliedrigen Ringes bedeuten kann, der bis zu 3 Heteroatome enthalten kann, wobei die Ringatome substituiert sein können und/oder C=C Doppelbindungen enthalten können,
Z Wasserstoff, Ammonium, Alkalimetallion, insbesondere Lithium, Natrium Kalium, 1/2 Äquivalente Erdalkalimetallion, vorzugsweise Calcium, Magnesium oder das Kation einer zur Neutralisation der freien Säuregruppe eingesetzte organische Stickstoffbase,
R¹⁰, R¹¹ unabhängig voneinander Wasserstoff, C₁-C₈-Alkyl oder C₅-C₁₀-Cycloalkyl und
n, m unabhängig voneinander Null oder 1 bedeuten.

2. Verbindungen nach Anspruch 1, worin R¹ bis R⁶ (einschließlich R^{4'}, R^{4"}, R^{5'} und R^{5"}) Wasserstoff bedeuten und n und m Null sind.

3. Verbindungen gemäß wenigstens einem der vorhergehenden Ansprüche, **dadurch gekennzeichnet, daß** sie wenigstens einer der folgenden Formeln Ia bis Ig entsprechen: 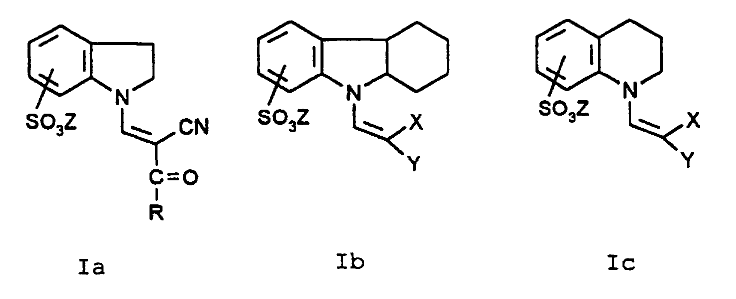 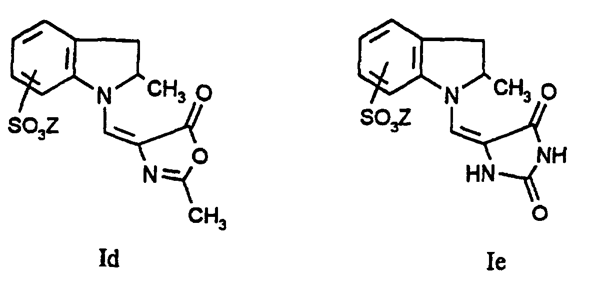 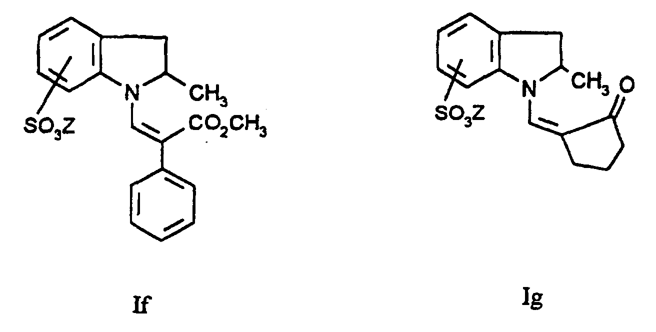 wobei die Substituenten die in Anspruch 1 angegebene Bedeutung aufweisen.

4. Verbindungen nach Anspruch 1, ausgewählt aus der Reihe bestehend aus
N-(β-Cyano-β-carbmethoxy-vinyl)-2-methylindolin-5-sulfonsäure,
N-(α-Methyl-β-β-dicarboethoxy-vinyl)-indolin-5-sulfonsäure,
N-(β-β-Dicarbmethoxy-vinyl)-2-methylindolin-5-sulfonsäure und
N-(β-Acetyl-β-carbomethoxy-vinyl)-2-methylindolin-5-sulfonsäure.

5. Verfahren zur Herstellung der Verbindungen nach Anspruch 1 durch Umsetzung von Verbindungen der Formel 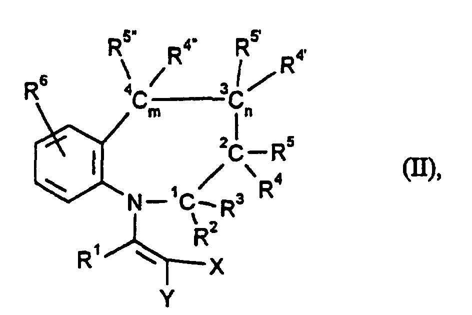 worin die Substituenten R¹ bis R⁶ (einschließlich R^{4'}, R^{4"}, R^{5'} und R^{5"}), X und Y und die Indices n und m die in Anspruch 1 angegebene Bedeutung besitzen, mit Sulfonierungsmitteln.

6. Verfahren nach Anspruch 5, wobei als Sulfonierungsmittel ein Schwefelsäure/Acetanhydrid-Gemisch eingesetzt wird.

7. Verfahren nach Anspruch 5, wonach man die Umsetzung in Essigsäure als Lösungsmittel durchführt.

8. Verwendung von Verbindungen (I) nach Anspruch 1 zur Verwendung als UV-Absorber.

9. Verbindungen nach Anspruch 8 zur Verwendung in Sonnenschutzmitteln.

## Claims

1. The invention relates to compounds of the formula 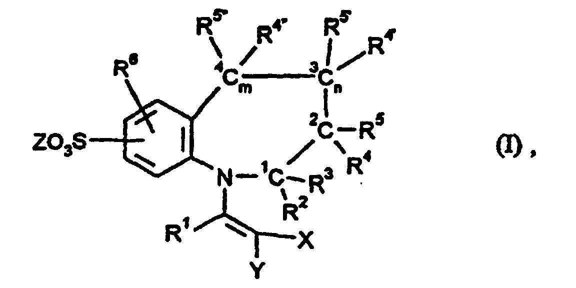 wherein
R¹ to R⁵ (including R^{4'}, R^{4"}, R^{5'}, R^{5"}) independently of one another denote hydrogen, C₁-C₈-alkyl or C₅-C₁₀-cycloalkyl, with the proviso that two substituents on adjacent C atoms from the series consisting of ¹C to ⁴C can also together denote an optionally substituted polymethylene group, wherein a methylene group can be replaced by -O-, -S- or -NH-,
R⁶ denotes hydrogen, C₁-C₈-alkyl, C₅-C₁₀-cycloalkyl, hydroxyl, C₁-C₈-alkoxy, COOR⁶⁰, CONR⁶¹R⁶²
R⁶⁰ to R⁶² independently of one another denote hydrogen or C₁-C₆-alkyl,
X, Y independently of one another denote hydrogen, CN, CO₂R¹⁰, CONR¹⁰R¹¹, COR¹⁰
wherein one of the radicals X or Y additionally can be a C₁-C₈-alkyl radical, a C₅-C₁₀-aryl radical or a heteroaryl radical having 5 to 6 ring atoms (of these 1 to 2 heteroatoms from the series consisting of N, O, S),
wherein furthermore X and Y or
R¹ together with one of the radicals X and Y can denote the radical to complete a 5 to 7-membered ring, which can contain up to 3 heteroatoms, wherein the ring atoms can be substituted and/or can contain C=C double bonds,
Z denotes hydrogen, ammonium, an alkali metal ion, in particular lithium, sodium potassium, 1/2 equivalent of an alkaline earth metal ion, preferably calcium, magnesium, or the cation of an organic nitrogen base employed for neutralization of the free acid group,
R¹⁰, R¹¹ independently of one another denote hydrogen, C₁-C₈-alkyl or C₅-C₁₀-cycloalkyl and
n, m independently of one another denote zero or 1.

2. Compounds according to claim 1, wherein R¹ to R⁶ (including R^{4'}, R^{4"}, R^{5'} and R^{5"}) denote hydrogen and n and m are zero.

3. Compounds according to at least one of the preceding claims, **characterized in that** they correspond to at least one of the following formulae Ia to Ig: 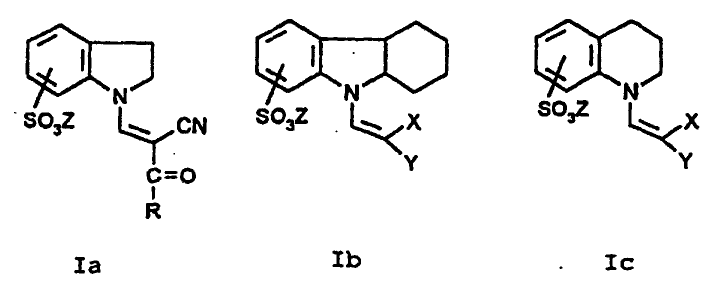 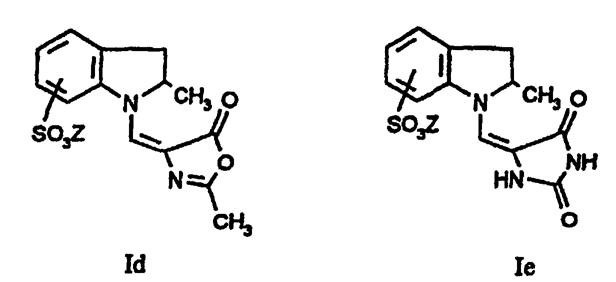 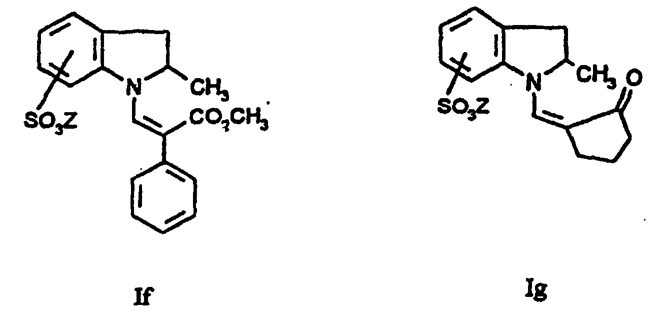 wherein the substituents have the meaning given in claim 1.

4. Compounds according to claim 1, chosen from the series consisting of
N-(β-cyano-β-carbmethoxy-vinyl)-2-methylindoline-5-sulfonic acid,
N-(α-methyl-β-β-dicarboethoxy-vinyl)-indoline-5-sulfonic acid,
N-(β-β-dicarbmethoxy-vinyl)-2-methylindoline-5-sulfonic acid and
N-(β-acetyl-β-carbomethoxy-vinyl)-2-methylindoline-5-sulfonic acid.

5. Process for the preparation of the compounds according to claim 1 by reaction of compounds of the formula 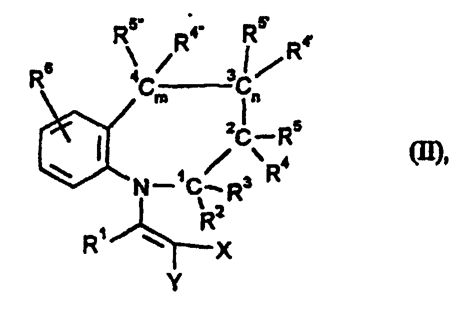 wherein the substituents R¹ to R⁶ (including R^{4'}, R^{4"}, R^{5'} and R^{5"}), X and Y and the indices n and m have the meaning given in claim 1, with sulfonating agents.

6. Process according to claim 5, wherein a sulfuric acid/acetic anhydride mixture is employed as the sulfonating agent.

7. Process according to claim 5, wherein the reaction is carried out in acetic acid as a solvent.

8. Use of compounds (I) according to claim 1 for use as UV absorbers.

9. Compounds according to claim 8 for use in sunscreen compositions.

## Revendications

1. Composés de formule 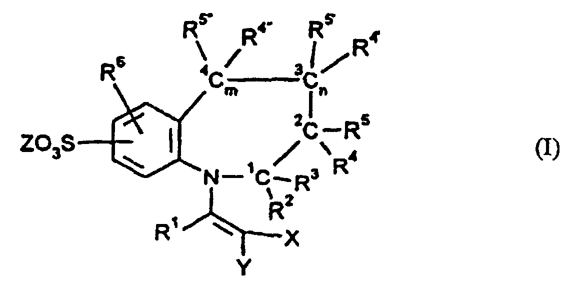 dans laquelle
R¹ à R⁵ (y compris R^{4'}, R^{4"}, R^{5'}, R^{5"}) représentent indépendamment les uns des autres un atome d'hydrogène ou un reste alkyle en C₁-C₈ ou cycloalkyle en C₅-C₁₀, à condition que deux substituants sur des atomes de carbone adjacents de la série des ¹C à ⁴C puissent aussi représenter ensemble un groupe polyméthylène éventuellement substitué dans lequel un groupe méthylène peut être remplacé par -O-, -S- ou -NH-,
R⁶ représente un atome d'hydrogène ou un reste alkyle en C₁-C₈, cycloalkyle en C₅-C₁₀, hydroxy, alcoxy en C₁-C₈, COOR⁶⁰, CONR⁶¹R⁶²,
R⁶⁰ à R⁶² représentent indépendamment les uns des autres un atome d'hydrogène ou un reste alkyle en C₁-C₆,
X, Y représentent indépendamment l'un de l'autre un atome d'hydrogène ou un groupe CN, CO₂R¹⁰, CONR¹⁰R¹¹ ou COR¹⁰,
l'un des restes X ou Y pouvant être en outre un reste alkyle en C₁-C₈, un reste aryle en C₅-C₁₀ ou un reste hétéroaryle de 5 à 6 chaînons cycliques (dont 1 à 2 hétéroatomes de la série N, O, S),
et, en outre, X et Y, ou R¹ avec l'un des restes X et Y, peuvent représenter le reste complétant un cycle de 5 à 7 chaînons pouvant contenir jusqu'à 3 hétéroatomes, les atomes cycliques pouvant être substitués, et/ou pouvant contenir des doubles liaisons C=C,
Z représente un atome d'hydrogène, un ammonium, un ion de métal alcalin, en particulier de lithium, de sodium ou de potassium, 1/2 équivalent d'ion de métal alcalino-terreux, de préférence de calcium ou de magnésium, ou le cation d'une base azotée organique utilisée pour la neutralisation du groupe acide libre,
R¹⁰, R¹¹ représentent, indépendamment l'un de l'autre, un atome d'hydrogène ou un reste alkyle en C₁-C₈ ou cycloalkyle en C₅-C₁₀, et
n, m sont indépendamment l'un de l'autre égaux à 0 ou 1.

2. Composés selon la revendication 1, dans lesquels R¹ à R⁶ (y compris R^{4'}, R^{4"}, R^{5'} et R^{5"}) représentent l'hydrogène et n et m sont égaux à 0.

3. Composés selon l'une au moins des revendications précédentes, **caractérisés en ce qu'**ils correspondent à au moins l'une des formules la à Ig ci-dessous: 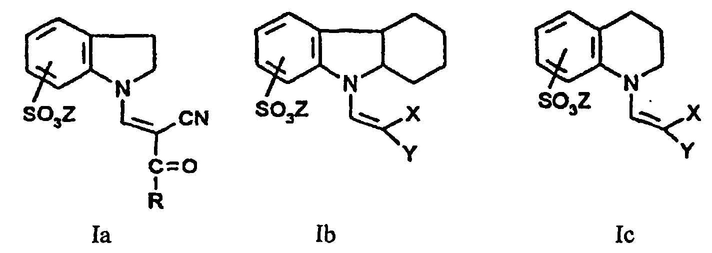 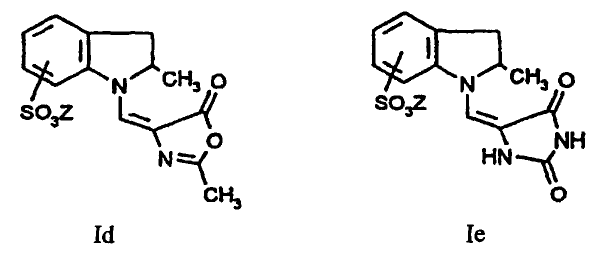 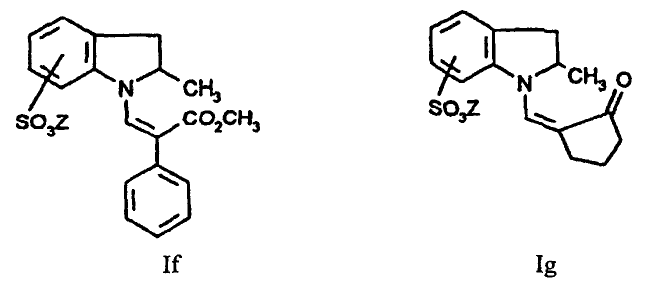 dans lesquelles les substituants ont la signification indiquée dans la revendication 1.

4. Composés selon la revendication 1, choisis dans la série constituée par
l'acide N-(β-cyano-β-carbométhoxyvinyl)-2-méthylindoline-5-sulfonique,
l'acide N-(α-méthyl-β,β-dicarbéthoxyvinyl)indoline-5-sulfonique,
l'acide N-(β,β-dicarbométhoxyvinyl)-2-méthylindoline-5-sulfonique et
l'acide N-(β-acétyl-β-carbométhoxyvinyl)-2-méthylindoline-5-sulfonique.

5. Procédé de préparation des composés selon la revendication 1 par réaction de composés de formule 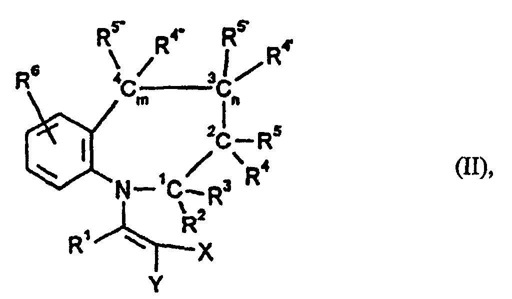 dans laquelle les substituants R¹ à R⁶ (y compris R^{4'}, R^{4"}, R^{5'} et R^{5"}), X et Y et les indices n et m ont la signification indiquée dans la revendication 1, avec des agents de sulfonation.

6. Procédé selon la revendication 5, dans lequel on utilise comme agent de sulfonation un mélange acide sulfurique/acétanhydride.

7. Procédé selon la revendication 5, selon lequel on effectue la réaction dans de l'acide acétique comme solvant.

8. Utilisation de composés (I) selon la revendication 1 pour une utilisation comme absorbants d'UV.

9. Composés selon la revendication 8 à utiliser dans des produits solaires.
